# EUROPEAN PATENT SPECIFICATION

(11) **EP 3 085 406 B1**
(45) Date of publication and mention of the grant of the patent: **17.03.2021**
(21) Application number: 16275063.2
(22) Date of filing: 25.04.2016
(51) Int. Cl.: A61M 25/02

(54) **APPARATUS FOR SECURING A DEVICE AND METHOD OF MANUFACTURING THE SAME**
VORRICHTUNG ZUR SICHERUNG EINER EINRICHTUNG UND VERFAHREN ZUR HERSTELLUNG DAVON
APPAREIL POUR FIXER UN DISPOSITIF ET PROCÉDÉ DE FABRICATION DE CELUI-CI

(30) Priority: 24.04.2015 GB 201507035
(43) Date of publication of application: 26.10.2016
(73) Proprietor: Optimum Medical Solutions Limited, Leeds, Yorkshire LS12 2LB (GB)
(72) Inventor: Rimmington, Gareth, Leeds LS12 2LB (GB)
(74) Representative: Corbyn, David Jonathan

(56) References cited:
- WO-A1-2010/002393
- WO-A1-2012/033456
- WO-A1-2014/036348
- WO-A1-2014/149668
- WO-A1-2015/031953
- WO-A1-2015/035238
- WO-A1-2015/134250
- WO-A2-2007/028007
- US-A1- 2011 118 670
- US-A1- 2014 330 247
- US-A1- 2015 038 912

## Description

The present invention relates to an apparatus suitable for the securing or attachment of a medical device to a location.

Although the following description refers to an apparatus for securing a catheter the skilled person will appreciate that the apparatus and method of the present invention could be used to secure other medical devices and tubing.

Clamps and anchors for securing tubing such as catheters to patients are known. Such clamps usually comprise an adhesive pad to which a clamp is mounted and within which the catheter tubing can be secured. The adhesive pad is usually attached to the patient's skin, for example on the thigh.

The problems with such clumps and anchors are that the area of skin to which the parties to be attached has to be cleaned before use and after use to remove any adhesive residue. Furthermore, the catheter tubing can move within the camp itself causing discomfort to the patient. Document US2015038912 discloses an apparatus according to the preamble of claim 1.

It is therefore an aim of the present invention to provide an apparatus that addresses the above-mentioned problems.

It is a further aim of the present invention to provide a method of manufacturing an apparatus that addresses the above-mentioned problems.

It is a yet further aim of the present invention to provide a method of using an apparatus that addresses the above-mentioned problems.

In a first aspect of the invention there is provided an apparatus according to claim 1.

Typically the apparatus secures a catheter to a surface in use. Further typically the surface is a skin surface, for example a patients thigh and/or the like.

Typically the silicone adhesive allows the apparatus to be attached to and/or removed from the surface or skin without preparation and/or cleaning of the same. Further typically the flexible layer or sheet includes a lower layer or sheet of silicone or substantially silicone based adhesive and/or silicone gel.

In one embodiment the adhesive is formed in a net or net arrangement. Typically the net arrangement includes strands or lines of adhesive between which there are pockets or pores. Further typically the adhesive is pressure sensitive adhesive.

In one embodiment the flexible layer or sheet is substantially transparent or translucent.

In one embodiment the flexible layer includes a release liner. Typically the release liner substantially covers the adhesive and is removed on use.

Typically the upper body portion includes side or lateral walls that at least partially define the side walls or the channel or conduit and a top wall connecting the side walls that at least partially defines a top wall or lid to the conduit. Further typically lower body portion the includes side or lateral walls that at least partially define the side walls or the channel or conduit and a bottom wall connecting the side walls that at least partially defines a bottom wall or base to the conduit.

In one embodiment the channel or conduit includes portion of deformable material. Typically the deformable material is a plastics material. Further typically the channel conduit includes one or more rings or lines of deformable material.

Typically, the deformable material provides one or more locations of increased resistance or grip portions to assist in securing the medical device in position in use.

In one embodiment the deformable material is a thermoplastic elastomer and/or the like.

In a preferred embodiment the lower body portion includes the deformable material. Typically the deformable material extends across the channel or conduit forming one or more ridges in the bottom wall and/or side walls.

Preferably the base portion is a substantially rectangular plate or surface.

In one embodiment the upper and lower body portions of the retainer member are pivotally connected. Typically the upper and lower body portions are connected via a hinge means or a hinged portion. Further typically the hinge means is located laterally or at an edge of the channel or conduit defined by the retainer member when in the closed condition.

Typically the hinge means is located between corresponding side walls of the upper and lower body portions.

In one embodiment the retainer member includes at least one catch or latch means. Typically the catchall latch means secures or selectively maintains the retainer member in the closed condition.

In one embodiment the catch or latch means includes one or more resiliently biased portions. Typically the latch means engages with one or more recesses or apertures to maintain the retainer member in the closed condition. Further typically the latch means is located on either the upper or lower body portion and the one or more recesses or apertures are located on the other of the top or bottom portion. Preferably the latch means is located on the top portion and the aperture is located on the bottom portion.

Typically the latch means and/or the aperture is located in or on corresponding side walls of the upper and lower body portions.

In one embodiment the latch means includes one or more actuating portions. Typically the actuating portion can be pressed by the user to disengage the latch means from the aperture and thereby open the retainer member. Further typically the latch means and/or aperture are located substantially at the side or laterally located in respect of the channel or conduit defined by the retainer member.

In one embodiment the upper and lower body portions define an entrance and/or exit apertures to the channel or conduit formed therein when in at least the closed condition. Typically at least the entrance aperture includes a substantially vertical post portion. Further typically the post portion divides the entrance aperture into two openings or apertures. As such this embodiment is particularly suitable for use with Foley catheters whereby the post portion is located between the drainage port and the balloon port or channel.

Preferably the conduit or channel is substantially rectangular in shape.

In a second aspect of the invention there is provided a method of manufacturing an apparatus according to claim 10.

In a third aspect of the invention there is provided a method of using an apparatus according to claim 11. References to embodiments throughout the description which are not under the scope of the appended claims merely represent possible exemplary executions and are therefore not part of the present invention.

Specific embodiments of the invention are now described with reference to the following figures wherein:
Figure 1a and 1b show perspective views of an apparatus in closed and open conditions respectively in accordance with one embodiment of the invention; and
Figure 2a and 2b show perspective views of an apparatus enclosing a catheter in closed and open conditions respectively in accordance with one embodiment of the invention.

The present invention relates to an improved anchor system for securing tubing to the skin of a patient and in particular catheters and Foley catheters.

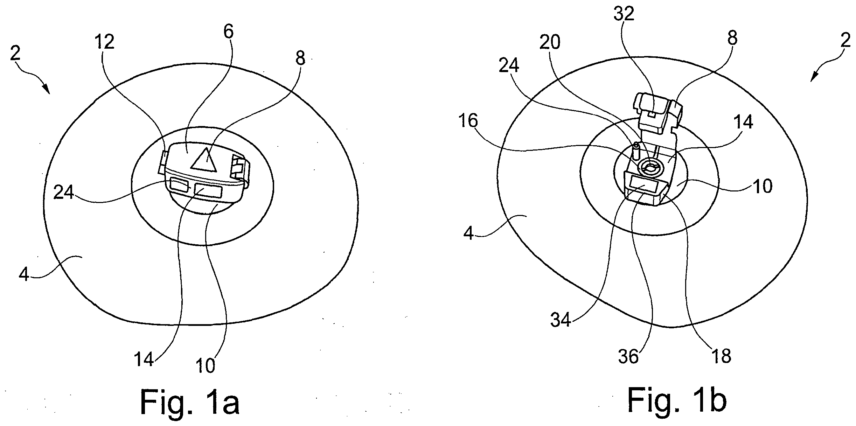

Figures 1a and 1b show an apparatus or device 2 particularly suited to securing Foley catheters in position on a patient. The device is shown in a closed condition or configuration in figure 1a and an open condition or configuration in figure 1b. The device 2 includes a flexible translucent layer or anchor pad 4 that incorporates a silicone adhesive to adhere or stick the anchor pad to the patient, typically on the thigh area. The anchor pad 4 is flexible and breathable to improve comfort. The anchor pad also remains adhered to the skin when wet, if for example the patient takes a shower. Centrally located on the anchor pad 4 is the retainer member or clamp 6. The clamp 6 comprises upper 8 and lower 10 body portions that are pivotally connected together via a hinge 12. The walls of the body portion define a channel 14 into which the catheter tubing is inserted and extends in use.

Typically the clamp or clip 6 body comprises polypropylene and/or the like.

The channel includes a deformable material 16 that extends across the lower body portion 10 that provides a deformable or flexible material to contact the catheter in use and provide an area of increased resistance to prevent movement and catheter slippage. Typically the material 16 is a thermoplastic elastomer and/or the like.

The device 2 in this example also includes a base member of plate 18 to which the clamp 6 is pivotally connected. As such, the clamp 6 can rotate or pivot about the central point or axis 20 of the lower body portion. As such, the catheter is secured in use to prevent any linear movement or slippage along the longitudinal axis of the catheter or channel but will move to accommodate any rotational movement. The rotational movement is substantially about an axis orthogonal to the longitudinal axis of the catheter or channel 14.

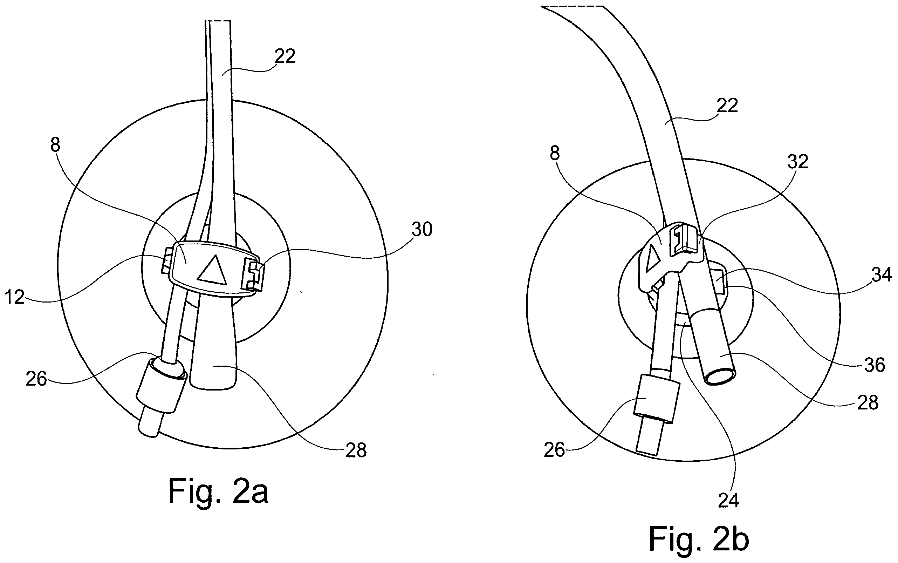

Figures 2a and 2b show the closed and open conditions respectively with a Foley catheter 22 inserted into position in the channel 14. In this embodiment the entrance to the clamp 6 includes a bar or post 24 that sits between the balloon port 26 and the fluid collection port 28 and further restrict any linear or longitudinal movement of the catheter tubing.

The clamp 6 in this example is secured in the closed position by a latching mechanism 30 that includes a deformable or resiliently biased latch member 32 formed on the side of the upper body portion 8 and which engages, with a sensory 'click', with an orifice or aperture 34 formed in the lower body portion 10. The resiliently biased latch has at least one formation or protrusion that engages with a further formation 36 or aperture formed in the orifice 34. In this example the latch 32 includes an actuating portion that is pressed in order to disengage the latching mechanism and thereby open the clamp 6 to remove or reposition the catheter 22.

## Claims

1. An apparatus for securing at least part of a catheter to a skin surface, said apparatus including a substantially flexible layer (4) having an adhesive lower surface and an upper surface, a retainer member (6) comprising upper (8) and lower (10) body portions and a base portion (18) located between the upper surface of the substantially flexible layer (4) and the lower body portion (10) of the retainer member (6), said retainer member (6) upper (8) and lower (10) body portions movable between an open condition and a closed condition whereby said body portions define at least one channel or conduit (14) within which at least part of a catheter is secured when in the closed condition, said flexible layer (4) is a polyurethane film and the adhesive lower layer includes silicone adhesive **characterised in that** the retainer (6) member is pivotally mounted on the base portion (18).

2. An apparatus according to claim 1 **characterised in that** the base portion (18) is more rigid or less flexible than the flexible layer (4).

3. An apparatus according to claim 1 **characterised in that** the adhesive is formed in a net arrangement of strands or lines of adhesive between which there are pockets.

4. An apparatus according to claim 1 **characterised in that** the flexible layer (4) is substantially transparent or translucent.

5. An apparatus according to claim 1 **characterised in that** the upper body portion (8) includes side or lateral walls that at least partially define the side walls or the channel or conduit (14) and a top wall connecting the side walls that at least partially defines a top wall or lid to the conduit.

6. An apparatus according to claim 5 **characterised in that** the lower body portion (10) the includes side or lateral walls that at least partially define the side walls or the channel or conduit (14) and a bottom wall connecting the side walls that at least partially defines a bottom wall or base to the conduit (14).

7. An apparatus according to claim 6 **characterised in that** the channel or conduit (14) includes portion of deformable plastics material (16) providing one or more locations of increased resistance or grip portions to assist in securing the catheter in position in use.

8. An apparatus according to claim 7 **characterised in that** the deformable material (16) is a thermoplastic elastomer included on the lower body portion (10), the deformable material extending across the channel or conduit forming one or more ridges in the bottom wall and/or side walls.

9. An apparatus according to claim 1 **characterised in that** the upper (8) and lower (10) body portions of the retainer member (6) are pivotally connected via a hinge means or a hinged portion (12) located laterally or at an edge of the channel or conduit (14) defined by the retainer member when in the closed condition.

10. A method of manufacturing an apparatus according to claim 1, **characterised in that** the method includes the step of pivotally mounting the retainer member (6) on the base portion (18).

11. A method of using an apparatus according to claim 1 for securing at least part of a catheter to a location, **characterised in that** the method includes the step of inserting at least part of the catheter in the channel or conduit and securing or closing the retainer member (6) in the closed condition.

## Patentansprüche

1. Vorrichtung zum Befestigen mindestens eines Teils eines Katheters an einer Hautoberfläche, wobei die genannte Vorrichtung eine im Wesentlichen flexible Schicht (4) mit einer klebenden Unterseite und einer Oberseite, ein Halteelement (6) mit einem oberen (8) und einem unteren (10) Körperabschnitt und einen Basisabschnitt (18) aufweist, der sich zwischen der Oberseite der im Wesentlichen flexiblen Schicht (4) und dem unteren Körperabschnitt (10) des Halteelements (6) befindet, wobei der obere (8) und untere (10) Körperabschnitt des genannten Halteelements (6) zwischen einem offenen Zustand und einem geschlossenen Zustand beweglich sind, so dass die genannten Körperabschnitte mindestens eine(n) Kanal oder Leitung (14) definieren, in dem/der im geschlossenen Zustand mindestens ein Teil eines Katheters befestigt ist, wobei die genannte flexible Schicht (4) ein Polyurethanfilm ist und die untere Klebeschicht einen Silikonklebstoff enthält, **dadurch gekennzeichnet, dass** das Halteelement (6) schwenkbar am Basisabschnitt (18) montiert ist.

2. Vorrichtung nach Anspruch 1, **dadurch gekennzeichnet, dass** der Basisabschnitt (18) steifer oder weniger flexibel ist als die flexible Schicht (4).

3. Vorrichtung nach Anspruch 1, **dadurch gekennzeichnet, dass** der Klebstoff in einer Netzanordnung von Klebstoffsträngen oder -linien ausgebildet ist, zwischen denen sich Taschen befinden.

4. Vorrichtung nach Anspruch 1, **dadurch gekennzeichnet, dass** die flexible Schicht (4) im Wesentlichen transparent oder transluzent ist.

5. Vorrichtung nach Anspruch 1, **dadurch gekennzeichnet, dass** der obere Körperabschnitt (8) seitliche oder laterale Wände aufweist, die zumindest teilweise die Seitenwände oder den Kanal oder die Leitung (14) definieren, und eine obere Wand, die die Seitenwände verbindet, die zumindest teilweise eine obere Wand oder einen Deckel für die Leitung definiert.

6. Vorrichtung nach Anspruch 5, **dadurch gekennzeichnet, dass** der untere Körperabschnitt (10) seitliche oder laterale Wände, die zumindest teilweise die Seitenwände oder den Kanal oder die Leitung (14) definieren, und eine die Seitenwände verbindende Bodenwand aufweist, die zumindest teilweise eine Bodenwand oder Basis für die Leitung (14) definiert.

7. Vorrichtung nach Anspruch 6, **dadurch gekennzeichnet, dass** der Kanal oder die Leitung (14) einen Abschnitt aus verformbarem Kunststoffmaterial (16) beinhaltet, der eine oder mehrere Stellen mit erhöhtem Widerstand oder Griffabschnitte bereitstellt, um die Befestigung des Katheters in seiner Position beim Gebrauch zu unterstützen.

8. Vorrichtung nach Anspruch 7, **dadurch gekennzeichnet, dass** das verformbare Material (16) ein thermoplastisches Elastomer ist, das auf dem unteren Körperabschnitt (10) enthalten ist, wobei sich das verformbare Material über den Kanal oder die Leitung erstreckt und eine oder mehrere Rippen in der Bodenwand und/oder den Seitenwänden bildet.

9. Vorrichtung nach Anspruch 1, **dadurch gekennzeichnet, dass** der obere (8) und untere (10) Körperabschnitt des Halteelements (6) über ein Scharnier oder einen Scharnierabschnitt (12) schwenkbar verbunden sind, das/der sich lateral oder an einem Rand des Kanals oder der Leitung (14) befindet, der/die im geschlossenen Zustand durch das Halteelement definiert wird.

10. Verfahren zur Herstellung einer Vorrichtung nach Anspruch 1, **dadurch gekennzeichnet, dass** das Verfahren den Schritt des schwenkbaren Montierens des Halteelements (6) am Basisabschnitt (18) beinhaltet.

11. Verfahren zur Verwendung einer Vorrichtung nach Anspruch 1 zum Befestigen zumindest eines Teils eines Katheters an einer Stelle, **dadurch gekennzeichnet, dass** das Verfahren den Schritt des Einführens zumindest eines Teils des Katheters in den Kanal oder die Leitung und des Befestigens oder Schließens des Halteelements (6) im geschlossenen Zustand beinhaltet.

## Revendications

1. Appareil pour fixer au moins une partie d'un cathéter à une surface de la peau, ledit appareil comportant une couche sensiblement flexible (4) ayant une surface inférieure adhésive et une surface supérieure, un élément de retenue (6) comprenant des parties de corps supérieure (8) et inférieure (10) et une partie de base (18) située entre la surface supérieure de la couche sensiblement flexible (4) et la partie de corps inférieure (10) de l'élément de retenue (6), lesdites parties de corps supérieure (8) et inférieure (10) de l'élément de retenue (6) étant mobiles entre un état ouvert et un état fermé, de sorte que lesdites parties de corps définissent au moins un canal ou conduit (14) dans lequel au moins une partie d'un cathéter est fixée lorsqu'il est à l'état fermé, ladite couche flexible (4) étant un film de polyuréthane et la couche inférieure adhésive comprenant un adhésif de silicone, **caractérisé en ce que** l'élément de retenue (6) est monté de manière pivotante sur la partie de base (18).

2. Appareil selon la revendication 1, **caractérisé en ce que** la partie de base (18) est plus rigide ou moins flexible que la couche flexible (4).

3. Appareil selon la revendication 1, **caractérisé en ce que** l'adhésif est formé selon un agencement en filet constitué de brins ou de lignes d'adhésif entre lesquels se trouvent des poches.

4. Appareil selon la revendication 1, **caractérisé en ce que** la couche flexible (4) est sensiblement transparente ou translucide.

5. Appareil selon la revendication 1, **caractérisé en ce que** la partie de corps supérieure (8) comporte des parois latérales qui définissent au moins partiellement les parois latérales ou le canal ou conduit (14) et une paroi supérieure reliant les parois latérales qui définit au moins partiellement une paroi supérieure ou un couvercle au conduit.

6. Appareil selon la revendication 5, **caractérisé en ce que** la partie de corps inférieure (10) comporte des parois latérales qui définissent au moins partiellement les parois latérales ou le canal ou conduit (14) et une paroi de fond reliant les parois latérales qui définit au moins partiellement une paroi de fond ou base au conduit (14).

7. Appareil selon la revendication 6, **caractérisé en ce que** le canal ou conduit (14) comprend une partie en matière plastique déformable (16) fournissant un ou plusieurs emplacements de résistance accrue ou des parties de préhension pour aider à fixer le cathéter en position lors de l'utilisation.

8. Appareil selon la revendication 7, **caractérisé en ce que** le matériau déformable (16) est un élastomère thermoplastique qui se trouve sur la partie de corps inférieure (10), le matériau déformable s'étendant à travers le canal ou conduit formant une ou plusieurs arêtes dans la paroi inférieure et/ou les parois latérales.

9. Appareil selon la revendication 1, **caractérisé en ce que** les parties de corps supérieure (8) et inférieure (10) de l'élément de retenue (6) sont reliées de manière pivotante par l'intermédiaire d'un moyen formant charnière ou d'une partie articulée (12) situé(e) latéralement ou sur un bord du canal ou conduit (14) défini par l'élément de retenue lorsqu'il se trouve à l'état fermé.

10. Procédé de fabrication d'un appareil selon la revendication 1, **caractérisé en ce que** le procédé comprend l'étape consistant à monter de manière pivotante l'élément de retenue (6) sur la partie de base (18).

11. Procédé d'utilisation d'un appareil selon la revendication 1 pour fixer au moins une partie d'un cathéter à un emplacement, **caractérisé en ce que** le procédé comprend l'étape consistant à insérer au moins une partie du cathéter dans le canal ou conduit et à fixer ou à fermer l'élément de retenue (6) à l'état fermé.
